# EUROPÄISCHE PATENTSCHRIFT

(11) **EP 4 216 873 B1**
(45) Veröffentlichungstag und Bekanntmachung des Hinweises auf die Patenterteilung: **24.07.2024**
(21) Anmeldenummer: 21777214.4
(22) Anmeldetag: 08.09.2021
(51) Int. Cl.: A61F 2/16

(54) **KOLBEN FÜR EINEN INJEKTOR UND INJEKTOR**
PLUNGER FOR AN INJECTOR, AND INJECTOR
PISTON POUR UN INJECTEUR ET INJECTEUR

(30) Priorität: 25.09.2020 DE 102020125130
(43) Veröffentlichungstag der Anmeldung: 02.08.2023
(73) Patentinhaber: Carl Zeiss Meditec AG, 07745 Jena (DE)
(72) Erfinder: MOEIN, Hadi, 73447 Oberkochen (DE)
(74) Vertreter: PATERIS Patentanwälte PartmbB
(86) Internationale Anmeldenummer: PCT/EP2021/074650
(87) Internationale Veröffentlichungsnummer: WO 2022/063573

(56) Entgegenhaltungen:
- EP-A1- 3 494 927
- WO-A1-2008/014260
- WO-A1-92/05816
- DE-T2- 69 635 279
- US-A- 2 717 599
- US-A- 5 772 666
- US-A1- 2014 066 946
- US-A1- 2014 257 316

## Beschreibung

Die Erfindung betrifft einen Kolben für einen Injektor zum Einführen einer Intraokularlinse in den Kapselsack eines Auges und den Injektor mit dem Kolben.

Bei der Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges z. B. mittels Phakoemulsifikation zerkleinert und anschließend aus dem Kapselsack des Auges abgesaugt. Danach wird eine Intraokularlinse in das Auge eingesetzt. Dabei wird die Intraokularlinse gefaltet, so dass sie durch die Spitze des Injektors passt. Die Spitze wird durch den Schnitt in den Kapselsack eingeführt und die gefaltete Intraokularlinse wird von einem Kolben des Injektors durch die Spitze in den Kapselsack geschoben, in dem die Intraokularlinse sich entfaltet und somit die ursprüngliche Linse ersetzt. Wenn der Kolben in die Spitze geschoben wird, wird er gequetscht, wodurch das Längsende des Kolbens seinen Querschnitt verkleinert. Nachteilig dabei ist jedoch, dass dabei die Spitze und der Kolben unter einer mechanischen Spannung stehen. Dies kann zu einer Beschädigung des Kolbens und/oder der Spitze führen.

DE 696 35 279 T2 offenbart ein Einspritzgerät mit einer deformierbaren Kolbenspitze für eine verformbare Intraokularlinse. US 2014/0257316 A1 offenbart eine Kartusche für einen Intraokularlinseninjektor. US 2,717,599 betrifft Verbesserungen in Kanülen für hypodermale Injektionen. WO 92/05816 A1 offenbart eine Hohlnadel zur medizinischen Verwendung. EP 3 494 927 A1 offenbart eine männliche Form als ein Anbringelement für einen Injektor.

Aufgabe der Erfindung ist es daher, einen einen Kolben aufweisenden Injektor zum Einführen einer Intraokularlinse in den Kapselsack eines Auges zu schaffen, bei dem die Wahrscheinlichkeit einer Beschädigung des Kolbens und/oder einer Spitze des Injektors gering ist.

Der erste erfindungsgemäße Injektor, der eingerichtet ist, eine Intraokularlinse in den Kapselsack eines Auges zu injizieren, weist auf: eine Spitze und einen Kolben, der ein verformbares Kissen aufweist, das einen ungequetschten Zustand und einen gequetschten Zustand hat und an einem Längsende des Kolbens eine Kontaktfläche aufweist, in die eine Aussparung eingebracht ist, wobei in dem ungequetschten Zustand die Kontaktfläche eine die Kontaktfläche außen begrenzende Außenkontur und eine die Aussparung außen begrenzende Innenkontur aufweist, wobei in der Aussparung ein gedachter Referenzpunk angeordnet ist, der der Mittelpunkt eines gedachten und in der Ebene der Kontaktfläche angeordneten Kreises ist, wobei der Kolben mindestens zwei Punktegruppen aufweist, die jeweils einen auf der Außenkontur angeordneten Auslegungspunkt, einen auf der Innenkontur angeordneten Innenkonturpunkt und den Referenzpunkt aufweisen, wobei für jede der Punktegruppen gilt, dass der Abstand von dem Auslegungspunkt zu dem Kreis gleich lang ist wie der Abstand von dem Innenkonturpunkt zu dem Referenzpunkt, dass der Auslegungspunkt, der Innenkonturpunkt und der Referenzpunkt auf einer Geraden liegen und dass der Innenkonturpunkt zwischen dem Auslegungspunkt und dem Referenzpunkt liegt, wobei der Abstand von dem Innenkonturpunkt zu dem Referenzpunkt für mindestens zwei der Punktegruppen unterschiedlich ist, wobei der Kolben in dem Injektor in einer Verschieberichtung längsverlagerbar gelagert ist und eingerichtet ist, mit der Kontaktfläche voran in die Spitze verschoben zu werden, wobei der Referenzpunkt so gewählt ist, dass der Referenzpunkt, wenn der Referenzpunkt in die Verschieberichtung projiziert ist, mit dem Schwerpunkt eines ersten inneren Querschnitts der Spitze zusammenfällt, dessen Normale parallel zu der Verschieberichtung ist.

Der zweite erfindungsgemäße Injektor, der eingerichtet ist, eine Intraokularlinse in den Kapselsack eines Auges zu injizieren, weist auf: eine Spitze und einen Kolben, der ein verformbares Kissen aufweist, das einen ungequetschten Zustand und einen gequetschten Zustand hat und an einem Längsende des Kolbens eine Kontaktfläche aufweist, in die eine Aussparung eingebracht ist, wobei in dem ungequetschten Zustand die Kontaktfläche eine die Kontaktfläche außen begrenzende Außenkontur und eine die Aussparung außen begrenzende Innenkontur aufweist, wobei in der Aussparung ein gedachter Referenzpunkt angeordnet ist, der der Mittelpunkt einer gedachten und in der Ebene der Kontaktfläche angeordneten geschlossenen Kurve ist, wobei der Kolben mindestens zwei Punktegruppen aufweist, die jeweils einen auf der Außenkontur angeordneten Auslegungspunkt, einen auf der Innenkontur angeordneten Innenkonturpunkt und den Referenzpunkt aufweisen, wobei für jede der Punktegruppen gilt, dass der Abstand von dem Auslegungspunkt zu der geschlossenen Kurve gleich lang ist wie der Abstand von dem Innenkonturpunkt zu dem Referenzpunkt, dass der Auslegungspunkt, der Innenkonturpunkt und der Referenzpunkt auf einer Geraden liegen und dass der Innenkonturpunkt zwischen dem Auslegungspunkt und dem Referenzpunkt liegt, wobei der Abstand von dem Innenkonturpunkt zu dem Referenzpunkt für mindestens zwei der Punktegruppen unterschiedlich ist, wobei der Kolben in dem Injektor in einer Verschieberichtung längsverlagerbar gelagert ist und eingerichtet ist, mit der Kontaktfläche voran in die Spitze verschoben zu werden, wobei die geschlossene Kurve in ihrem Inneren eine Fläche begrenzt, die die gleiche Form hat wie ein zweiter innerer Querschnitt der Spitze an deren engster Stelle, wobei die Normale des zweiten inneren Querschnitts parallel zu der Verschieberichtung ist. Der Mittelpunkt ist der Schwerpunkt der von der geschlossenen Kurve in ihrem Inneren begrenzten Fläche.

Bei einem Betrieb des Injektors ist der Kolben von einem Ort außerhalb der Spitze in Richtung zu der Spitze hin zu verlagern. Wird der Kolben in Richtung zu der Spitze hin verlagert, befindet sich das Kissen zuerst in dem ungequetschten Zustand. Die Kontaktfläche berührt die Intraokularlinse und schiebt die Intraokularlinse zu der Spitze hin. Gelangt der Kolben während einer Vorwärtsbewegung in die Spitze, so wird er gequetscht und das Kissen gelangt dadurch in einen teilweise gequetschten Zustand. Dabei verändert sich die Form der Kontaktfläche und das Volumen der Aussparung wird mit zunehmender Vorwärtsbewegung kleiner. Indem das Volumen der Aussparung kleiner wird, können mechanische Spannungen sowohl in dem Kolben als auch in der Spitze verringert werden, wodurch eine Wahrscheinlichkeit einer Beschädigung des Kolbens und der Spitze verringert werden können. Indem die Innenkontur erfindungsgemäß ausgelegt ist, kann eine räumliche Verteilung der mechanischen Spannungen in dem Kissen und in der das Kissen umgebenden Spitze vergleichmäßigt werden. Dadurch können lokale Maxima der mechanischen Spannungen verringert werden, wodurch die Wahrscheinlichkeit der Beschädigung des Kissens und der Spitze besonders niedrig ist. Wird die Kontaktfläche bis zu einer Öffnung der Spitze verlagert, an dem die Intraokularlinse den Injektor verlässt, so kann das Kissen nicht weiter gequetscht werden und das Kissen befindet sich in dem gequetschten Zustand. Dabei ist denkbar, dass das Kissen in dem Bereich der Kontaktfläche in dem gequetschten Zustand außen im Wesentlichen die Form des Kreises oder der geschlossenen Kurve hat.

Es ist bevorzugt, dass der Kolben mindestens drei der Punktegruppen aufweist. Dadurch kann die räumliche Verteilung der mechanischen Spannungen in dem Kissen besonders vergleichmäßigt werden. Es ist besonders bevorzugt, dass der Abstand von dem Innenkonturpunkt zu dem Referenzpunkt für mindestens drei der Punktegruppen unterschiedlich ist. Dadurch kann die räumliche Verteilung der mechanischen Spannungen noch weiter vergleichmäßigt werden.

Die Außenkontur hat bevorzugt eine von einem Kreis verschiedene Form. Es ist zudem denkbar, dass die Innenkontur eine von einem Kreis verschiedene Form hat. Es ist bevorzugt, dass die Innenkontur eine Interpolation der Innenkonturpunkte ist. Die Interpolation kann beispielsweise einen Spline oder eine Linie aufweisen. Der Spline oder die Linie können zwei benachbarte Innenkonturpunkte miteinander verbinden.

Es ist bevorzugt, dass der Kreis 8 einen Durchmesser von 1,4 mm bis 3,0 mm hat. Zudem ist bevorzugt, dass zwei auf der Außenkontur 7 angeordnete Punkte maximal einen Abstand von 4,0 mm, insbesondere von 3,5 mm, haben. Dabei gilt die Maßgabe, dass der Durchmesser des Kreises und die Form der Außenkontur in dem ungequetschten Zustand so zu wählen sind, dass der Kreis vollständig innerhalb der Außenkontur liegt. Es ist zudem denkbar, dass zwei benachbarte Außenkonturpunkte einen Winkelabstand von maximal 120° haben.

Das Kissen weist bevorzugt einen sich erweiternden Abschnitt auf, dessen Querschnitt sich in Richtung zu der Kontaktfläche hin erweitert. Dadurch hat das Kissen ein geringeres Volumen als in dem Fall, dass das Kissen entlang der Länge des sich erweiternden Bereichs die Dicke der Außenkontur hätte. Dadurch können mechanische Spannungen in dem Kissen und in der Spitze beim Quetschen des Kissens verringert werden. Es ist besonders bevorzugt, dass die Aussparung sich in dem sich erweiternden Abschnitt erstreckt und sich in dem sich erweiternden Abschnitt in einer Richtung weg von der Kontaktfläche verjüngt. Dadurch ist es möglich, eine Dicke des Kissens in einer Längsrichtung des Kolbens konstant zu halten.

Es ist bevorzugt, dass das Kissen einen vorstehenden Abschnitt aufweist, der quer zu einer Richtung, die parallel zu der Normalen der Kontaktfläche ist, von dem verbliebenen Kissen vorsteht und die Kontaktfläche bildet.

Es ist für den zweiten erfindungsgemäßen Injektor bevorzugt, dass der Referenzpunkt so gewählt ist, dass der Referenzpunkt, wenn der Referenzpunkt in die Verschieberichtung projiziert ist, mit dem Schwerpunkt eines ersten inneren Querschnitts der Spitze zusammenfällt, dessen Normale parallel zu der Verschieberichtung ist. Dadurch wird erreicht, dass das Kissen, wenn es in die Spitze geschoben wird, möglichst gleichmäßig entlang seines Umfangs gequetscht wird. Dadurch kann die Verteilung der mechanischen Spannungen besonders vergleichmäßigt werden.

Der Kreis hat bevorzugt den gleichen Durchmesser wie ein zweiter innerer Querschnitt der Spitze an deren engster Stelle, wobei die Normale des zweiten inneren Querschnitts parallel zu der Verschieberichtung ist. Die geschlossene Kurve begrenzt erfindungsgemäß in ihrem Inneren eine Fläche, die die gleiche Form hat wie ein zweiter innerer Querschnitt der Spitze an deren engster Stelle, wobei die Normale des zweiten inneren Querschnitts parallel zu der Verschieberichtung ist. Dabei ist denkbar, dass der erste innere Querschnitt und der zweite innere Querschnitt zusammenfallen. Zudem ist denkbar, dass der erste innere Querschnitt und der zweite innere Querschnitt an der Öffnung der Spitze angeordnet sind.

Im Folgenden wird die Erfindung anhand der beigefügten schematischen Zeichnungen näher erläutert.
Figur 1 zeigt eine Draufsicht auf eine erste Ausführungsform eines Kolbens.
Figur 2 zeigt eine Draufsicht auf eine zweite Ausführungsform des Kolbens.
Figur 3 zeigt eine erste perspektivische Ansicht der ersten Ausführungsform.
Figur 4 zeigt eine zweite perspektivische Ansicht der ersten Ausführungsform.
Figur 5 zeigt eine Draufsicht auf einen Injektor.
Figur 6 zeigt einen Injektor in einer schematischen Seitenansicht.

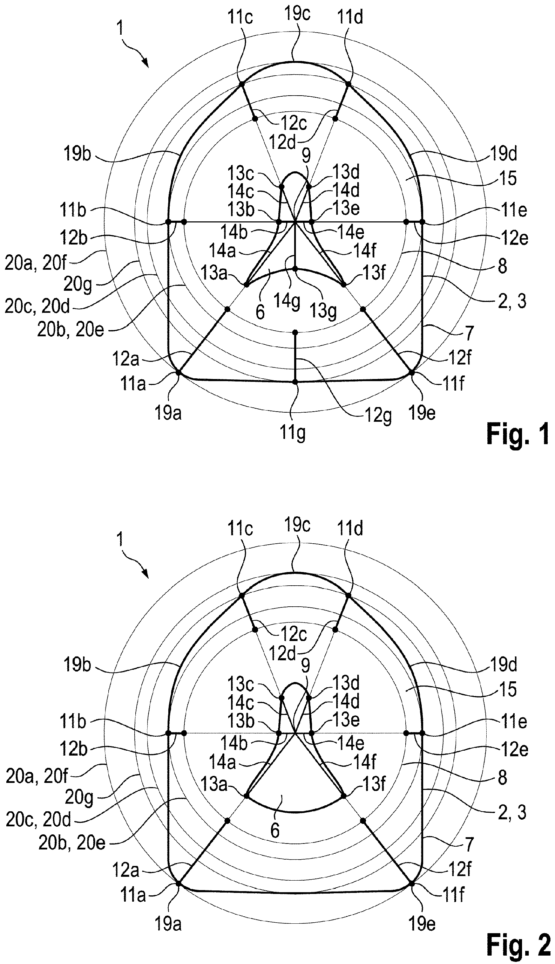

Wie es aus Figuren 1 bis 5 ersichtlich ist, weist ein Kolben 1 für einen Injektor 17, der eingerichtet ist, eine Intraokularlinse 22 in den Kapselsack eines Auges zu injizieren, ein verformbares Kissen 2 auf, das einen ungequetschten Zustand und einen gequetschten Zustand einnehmen kann. Das Kissen 2 weist an einem Längsende des Kolbens 1 eine Kontaktfläche 15 auf, in die eine Aussparung 6 eingebracht ist, wobei in dem ungequetschten Zustand die Kontaktfläche 15 eine die Kontaktfläche 15 außen begrenzende Außenkontur 7 und eine die Aussparung 6 außen begrenzende Innenkontur 10 aufweist. In der Aussparung 6 ist ein gedachter Referenzpunkt 9 angeordnet, der der Mittelpunkt eines gedachten und in der Ebene der Kontaktfläche 15 angeordneten Kreises 8 oder einer anderen geschlossenen Kurve ist, wobei der Kolben 1 mindestens zwei Punktegruppen aufweist. Die Punktegruppen weisen jeweils einen auf der Außenkontur 7 angeordneten Auslegungspunkt 11, einen auf der Innenkontur 10 angeordneten Innenkonturpunkt 13 und den Referenzpunkt 9 auf, wobei für jede der Punktegruppen gilt, dass der Abstand 12 von dem Auslegungspunkt 11 zu dem Kreis 8 oder der geschlossenen Kurve gleich lang ist wie der Abstand 14 von dem Innenkonturpunkt 13 zu dem Referenzpunkt 9. Ferner gilt für jede der Punktegruppen, dass der Auslegungspunkt 11, der Innenkonturpunkt 13 und der Referenzpunkt 9 auf einer Geraden liegen und dass der Innenkonturpunkt 13 zwischen dem Auslegungspunkt 11 und dem Referenzpunkt 9 liegt, wobei der Abstand 14 von dem Innenkonturpunkt 13 zu dem Referenzpunkt 9 für mindestens zwei der Punktegruppen unterschiedlich ist. In dem Fall, dass die geschlossene Kurve vorgesehen ist, ist der Mittelpunkt der Schwerpunkt der von der geschlossenen Kurve in ihrem Inneren begrenzten Fläche.

In den Figuren 1 und 2 sind mehrere Punktegruppen eingezeichnet, welche jeweils mit der Endung "a", "b", "c", "d", "e" "f" oder "g" versehen sind. Die erste Punktegruppe weist einen Auslegungspunkt 11a auf. Wird zwischen dem Auslegungspunkt 11a und dem Referenzpunkt 9 eine direkte gerade Linie gezogen, so ergibt sich zwischen dem Auslegungspunkt 11a und dem Kreis 8 oder der geschlossenen Kurve eine Strecke mit einem Abstand 12a. Dieser Abstand 12a wird vom Referenzpunkt 9 ausgehend in Richtung zum Auslegungspunkt 11a angelegt, sodass sich Innenkonturpunkt 13a ergibt. Die zwischen dem Referenzpunkt 9 und dem Innenkonturpunkt 13a erreichte Streckenlänge, welche mit 14a bezeichnet ist, besitzt somit den gleichen Betrag wie der Abstand 12a.

Die zweite Punktegruppe weist den Auslegungspunkt 11b und Innenkonturpunkt 13b auf, wobei der Abstand zwischen dem Auslegungspunkt 11b und dem Kreis 8 oder der geschlossenen Kurve mit 12b bezeichnet ist. Dieser Abstand 12b besitzt den gleichen Betrag wie der Abstand 14b, welcher zwischen dem Referenzpunkt 9 und dem Innenkonturpunkt 13b vorliegt. Die dritte Punktegruppe weist den Auslegungspunkt 11c und den Innenkonturpunkt 13c auf, zu welchen die Abstände 12c und 14c gehören. Die vierte Punktegruppe weist den Auslegungspunkt 11d und den Innenkonturpunkt 13d auf, zu welchen die Abstände 12d und 14d gehören. Die fünfte Punktegruppe weist den Auslegungspunkt 11e und Innenkonturpunkt 13e mit den Abständen 12e und 14e auf. Die sechste Punktegruppe weist den Auslegungspunkt 11f und Innenkonturpunkt 13f mit den Abständen 12f und 14f auf. Die siebte Punktegruppe weist den Auslegungspunkt 11g und Innenkonturpunkt 13g mit den Abständen 12g und 14g auf.

Figuren 1 und 2 zeigen zudem, dass in dem ungequetschten Zustand des Kissens 2 der Kreis 8 oder die geschlossenen Kurve vollständig innerhalb der Außenkontur 7 angeordnet sein kann. Zudem ist in Figuren 1 und 2 für jede der Punktegruppen ein Kreis 20 eingezeichnet, dessen Mittelpunkt der Referenzpunkt 9 ist und der durch den jeweiligen Auslegungspunkt 11 der jeweiligen Punktegruppe verläuft. Demnach ist für die erste Punktegruppe ein erster Kreis 20a und für die zweite Punktegruppe ein zweiter Kreis 20b eingezeichnet. Für die dritte Punktegruppe ist ein Kreis 20c, für die vierte Punktegruppe ein Kreis 20d, für die fünfte Punktegruppe ein Kreis 20e, für die sechste Punktegruppe ein Kreis 20f und für die siebte Punktegruppe ein Kreis 20g eingezeichnet.

Figuren 1 bis 5 zeigen, dass die Außenkontur 7 eine von einem Kreis verschiedene Form haben kann. Zudem kann ebenfalls die Innenkontur 10 eine von einem Kreis verschiedene Form haben. Insbesondere Figuren 1 und 2 zeigen, dass die Kontaktfläche 15 beispielsweise die Form eines Fünfecks haben kann. Vier Ecken 19a, 19b, 19d und 19e der fünf Ecken 19a bis 19e können dabei in den Ecken eines gedachten Rechtecks angeordnet sein. Die fünfte Ecke 19c kann auf einer Gerade liegen, die zwei parallel angeordnete Seiten des Rechtsecks in deren Mittelpunkten schneidet. Zudem ist denkbar, dass die Ecken 19a bis 19e abgerundet sind, wie es auch insbesondere in den Figuren 1 und 2 dargestellt ist. Dadurch, dass die Ecken 19a bis 19e abgerundet sind, wird ein Gleiten des Kolbens 1 in dem Injektor 17 erleichtert. Zumindest ein Teil der Auslegungspunkte 11 kann in zumindest einem Teil der Ecken 19 angeordnet sein. Figuren 1 und 2 zeigen zudem, dass die Auslegungspunkte 11 symmetrisch bezüglich einer Spiegelachse der Kontaktfläche 15 angeordnet sein können.

Figuren 1 und 2 zeigen, dass der Kolben 1 mindestens drei der Punktegruppen aufweisen kann. Eine dritte der Punktegruppen weist einen dritten Auslegungspunkt 11c, einen dritten Innenkonturpunkt 13c und den Referenzpunkt 9 auf. Zudem ist denkbar, dass der Abstand 14 von dem Innenkonturpunkt 13 zu dem Referenzpunkt 9 für mindestens drei der Punktegruppen unterschiedlich ist. Figur 1 zeigt eine erste Ausführungsform des Kolbens 1 und Figur 2 zeigt eine zweite Ausführungsform des Kolbens 1, wobei bei der ersten Ausführungsform sieben der Auslegungspunkte 11a bis 11g vorgesehen sind und bei der zweiten Ausführungsform sechs der Auslegungspunkte 11a bis 11f vorgesehen sind.

Die Innenkontur kann eine Interpolation der Innenkonturpunkte 13 sein. Die Interpolation kann einen Spline oder eine Linie aufweisen.

Der Kreis 8 kann beispielsweise einen Durchmesser von 1,4 mm bis 3,0 mm haben. Zwei auf der Außenkontur 7 angeordneten Punkte können beispielsweise maximal einen Abstand von 4,0 mm, insbesondere von 3,5 mm, zueinander haben. Dabei gilt die Maßgabe, dass der Kreis 8 oder die geschlossene Kurve vollständig innerhalb der Außenkontur 7 angeordnet ist.

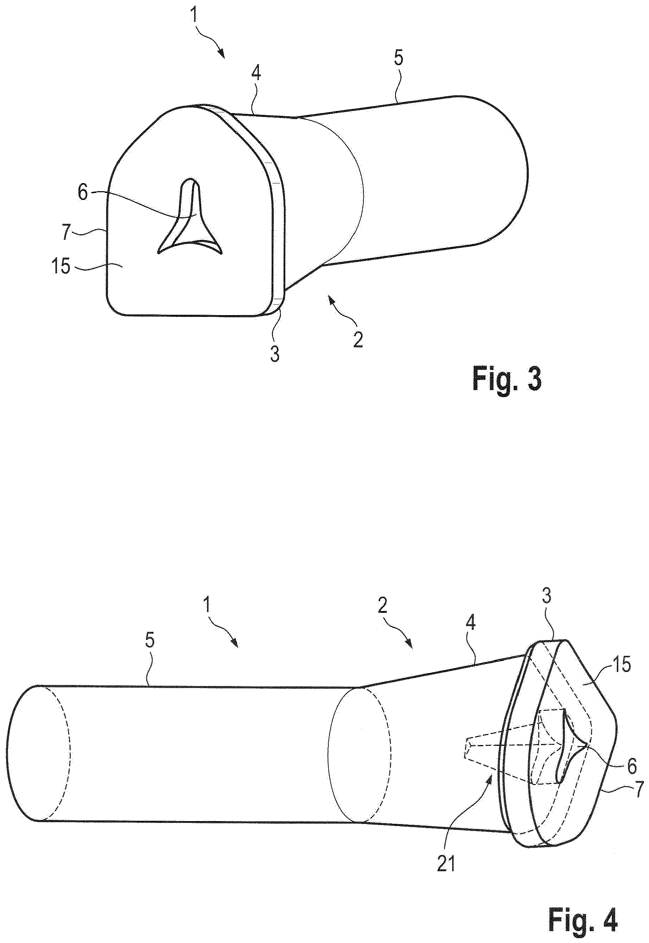

Wie es aus Figuren 3 und 4 ersichtlich ist, kann das Kissen 2 einen sich erweiternden Abschnitt 4 aufweisen, dessen LängsQuerschnitt sich in Richtung zu der Kontaktfläche 15 hin erweitert. Die Aussparung 6 kann sich in dem sich erweiternden Abschnitt 4 erstrecken und sich in dem sich erweiternden Abschnitt 4 in einer Richtung weg von der Kontaktfläche 15 verjüngen, siehe Bezugszeichen 21. Dabei kann in einer Ebene, die parallel beabstandet von der Kontaktfläche 15 angeordnet ist und durch die Aussparung 6 in dem sich erweiternden Abschnitt 4 verläuft, gelten, dass in dem ungequetschten Zustand das Kissen 2 eine das Kissen 2 außen begrenzende zweite Außenkontur und eine die Aussparung 6 außen begrenzende zweite Innenkontur aufweist, wobei in der Aussparung 6 ein gedachter zweiter Referenzpunkt angeordnet ist, der der Mittelpunkt eines gedachten und in der Ebene angeordneten zweiten Kreises 8 oder einer zweiten anderen geschlossenen Kurve ist, wobei der Kolben 1 mindestens zwei weitere Punktegruppen aufweist, die jeweils einen auf der zweiten Außenkontur angeordneten Auslegungspunkt, einen auf der zweiten Innenkontur angeordneten Innenkonturpunkt und den zweiten Referenzpunkt aufweisen, wobei für jede der weiteren Punktegruppen gilt, dass der Abstand von dem Auslegungspunkt zu dem zweiten Kreis oder der zweiten Kurve gleich lang ist wie der Abstand von dem Innenkonturpunkt zu dem zweiten Referenzpunkt, dass der Auslegungspunkt, der Innenkonturpunkt und der zweite Referenzpunkt auf einer Geraden liegen und dass der Innenkonturpunkt zwischen dem Auslegungspunkt und dem zweiten Referenzpunkt liegt, wobei der Abstand von dem Innenkonturpunkt zu dem zweiten Referenzpunkt für mindestens zwei der weiteren Punktegruppen unterschiedlich ist. Es ist zudem ersichtlich, dass der Kolben 2 einen zylinderförmigen Abschnitt 5 aufweisen kann, der an den sich erweiternden Abschnitt 4 an dessen der Kontaktfläche 15 abgewandten Ende angrenzt.

Figuren 3 und 4 zeigen zudem, dass das Kissen 2 einen vorstehenden Abschnitt 3 aufweisen kann, der quer zu einer Richtung, die parallel zu der Normalen der Kontaktfläche 15 ist, von dem verbliebenen Kissen 2 vorsteht und die Kontaktfläche 15 bildet. Der vorstehende Abschnitt 3 kann an den sich erweiternden Abschnitt 4 unmittelbar angrenzen.

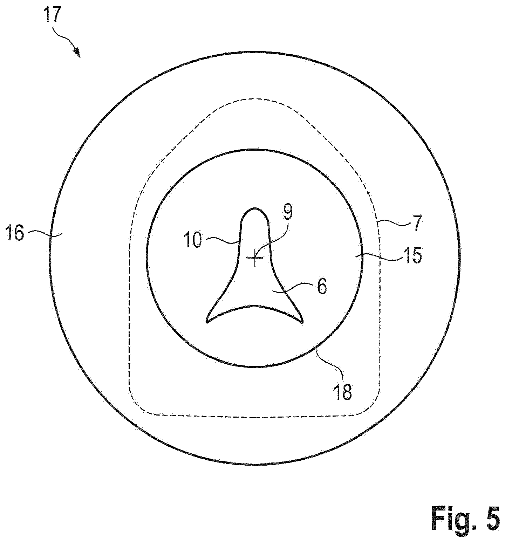

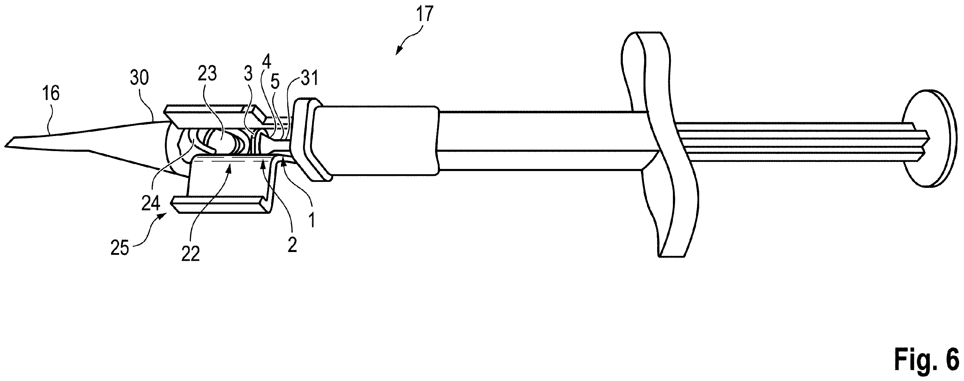

Figur 5 und 6 zeigen den Injektor 17 mit dem Kolben 1 und einer konisch sich verjüngenden Spitze 16, wobei der Kolben 1 in dem Injektor 17 in einer Verschieberichtung längsverlagerbar gelagert ist und eingerichtet ist, mit der Kontaktfläche 15 voran in die Spitze 16 verschoben zu werden. Die Verschieberichtung steht dabei in Figur 5 senkrecht auf der Zeichenebene. Figur 5 und 6 zeigen zudem, dass sich die Spitze 16 in der Verschieberichtung hin zu einer Öffnung 18 der Spitze 16 verjüngt, wobei die Intraokularlinse 22 via die Öffnung 18 von dem Kolben 1 aus dem Injektor 17 heraus zu schieben ist. Es ist denkbar, dass der Referenzpunkt 9 so gewählt ist, dass der Referenzpunkt 9, wenn der Referenzpunkt 9 in die Verschieberichtung projiziert ist, mit dem Schwerpunkt eines ersten inneren Querschnitts der Spitze 16 zusammenfällt, dessen Normale parallel zu der Verschieberichtung ist. Es ist auch denkbar, dass der Kreis 8 den gleichen Durchmesser hat wie ein zweiter innerer Querschnitt der Spitze 16 an deren engster Stelle, wobei die Normale des zweiten inneren Querschnitts parallel zu der Verschieberichtung ist. Die engste Stelle kann dabei an der Öffnung 18 liegen und der erste innere Querschnitt und/oder der zweite innere Querschnitt können der Querschnitt der Öffnung 18 sein. Der erste innere Querschnitt und der zweite innere Querschnitt können zusammenfallen und die Öffnung 18 beinhalten. In dem Fall, dass die geschlossenen Kurve vorgesehen ist, kann die Form der von geschlossenen Kurve in ihrem Inneren begrenzten Fläche identisch sein mit dem zweiten inneren Querschnitt. Figur 6 zeigt zudem, dass die Außenkontur 7 in ihrem ungequetschten Zustand größer als der zweite Querschnitt sein kann.

Aus Figur 6 ist ersichtlich, dass die Intraokularlinse 22 einen Optikkörper 23 und eine Haptik 24 aufweisen kann. Die Intraokularlinse 22 ist dabei in einer Kassette 25 gehalten, welche in einem Injektorkörper 30 eingesetzt ist. Der Kolben 1 ist an einem Ende eines Stößels 31 angebracht, der linear im Injektorkörper 30 angebracht ist.

Vor einem Gebrauch des Injektors 17 befindet sich das Kissen 2 in dem ungequetschten Zustand. Während des Gebrauchs des Injektors 17 wird der Kolben 1 in die Spitze 16 hinein verlagert. Dabei kontaktiert die Kontaktfläche 15 die Intraokularlinse 22 und der Kolben 1 verschiebt die Intraokularlinse 22 in Richtung zu der Öffnung 18 hin. Das Kissen 2 stößt in der Spitze 16 an die Wand der Spitze 16 an. Durch ein weiteres Verlagern des Kolbens 1 zu der Öffnung 18 hin wird das Kissen 2 gequetscht, wodurch das Kissen 2 in einen teilweise gequetschten Zustand gelangt und die Aussparung 6 kleiner wird. Befindet sich die Kontaktfläche 15 in der Öffnung 18, so befindet sich das Kissen 2 in dem gequetschten Zustand. In dem gequetschten Zustand kann die äußere Kontur des Kissens 2 im Bereich der Kontaktfläche 15 im Wesentlichen die Form des Kreises 8 haben.

### Bezugszeichenliste

1 Kolben
2 Kissen
3 vorstehender Abschnitt
4 erweiternder Abschnitt
5 zylinderförmiger Abschnitt
6 Aussparung
7 Außenkontur
8 Kreis
9 Referenzpunkt
10 Innenkontur
11x x-ter Auslegungspunkt
12x Abstand des x-ten Auslegungspunkts dem Kreis 8
13x x-ter Innenkonturpunkt
14x Abstand des x-ten Innenkonturpunkt zum Referenzpunkt 9
15 Kontaktfläche
16 Spitze
17 Injektor
18 Öffnung
19x x-te Ecke
20a x-ter Kreis
21 Verjüngung der Aussparung
22 Intraokularlinse
23 Optikkörper
24 Haptik
25 Kassette
30 Injektorkörper
31 Stößel

## Patentansprüche

1. Injektor mit einem Kolben (1) und einer Spitze (16), wobei der Injektor (17) eingerichtet ist, eine Intraokularlinse (22) in den Kapselsack eines Auges zu injizieren, wobei der Kolben (1) ein verformbares Kissen (2) aufweist, das einen ungequetschten Zustand und einen gequetschten Zustand hat und an einem Längsende des Kolbens (1) eine Kontaktfläche (15) aufweist, in die eine Aussparung (6) eingebracht ist, wobei in dem ungequetschten Zustand die Kontaktfläche (15) eine die Kontaktfläche (15) außen begrenzende Außenkontur (7) und eine die Aussparung (6) außen begrenzende Innenkontur (10) aufweist, wobei in der Aussparung (6) ein gedachter Referenzpunkt (9) angeordnet ist, der der Mittelpunkt eines gedachten und in der Ebene der Kontaktfläche (15) angeordneten Kreises (8) ist, wobei der Kolben (1) mindestens zwei Punktegruppen aufweist, die jeweils einen auf der Außenkontur (7) angeordneten Auslegungspunkt (11), einen auf der Innenkontur (10) angeordneten Innenkonturpunkt (13) und den Referenzpunkt (9) aufweisen, wobei für jede der Punktegruppen gilt, dass der Abstand (12) von dem Auslegungspunkt (11) zu dem Kreis (8) gleich lang ist wie der Abstand (14) von dem Innenkonturpunkt (13) zu dem Referenzpunkt (9), dass der Auslegungspunkt (11), der Innenkonturpunkt (13) und der Referenzpunkt (9) auf einer Geraden liegen und dass der Innenkonturpunkt (13) zwischen dem Auslegungspunkt (11) und dem Referenzpunkt (9) liegt, wobei der Abstand (14) von dem Innenkonturpunkt (13) zu dem Referenzpunkt (9) für mindestens zwei der Punktegruppen unterschiedlich ist, wobei der Kolben (1) in dem Injektor (17) in einer Verschieberichtung längsverlagerbar gelagert ist und eingerichtet ist, mit der Kontaktfläche (15) voran in die Spitze (17) verschoben zu werden, wobei der Referenzpunkt (9) so gewählt ist, dass der Referenzpunkt (9), wenn der Referenzpunkt (9) in die Verschieberichtung projiziert ist, mit dem Schwerpunkt eines ersten inneren Querschnitts der Spitze (16) zusammenfällt, dessen Normale parallel zu der Verschieberichtung ist.

2. Injektor mit einem Kolben (1) und einer Spitze (16), wobei der Injektor (17) eingerichtet ist, eine Intraokularlinse (22) in den Kapselsack eines Auges zu injizieren, wobei der Kolben (1) ein verformbares Kissen (2) aufweist, das einen ungequetschten Zustand und einen gequetschten Zustand hat und an einem Längsende des Kolbens (1) eine Kontaktfläche (15) aufweist, in die eine Aussparung (6) eingebracht ist, wobei in dem ungequetschten Zustand die Kontaktfläche (15) eine die Kontaktfläche (15) außen begrenzende Außenkontur (7) und eine die Aussparung (6) außen begrenzende Innenkontur (10) aufweist, wobei in der Aussparung (6) ein gedachter Referenzpunkt (9) angeordnet ist, der der Mittelpunkt einer gedachten und in der Ebene der Kontaktfläche (15) angeordneten geschlossenen Kurve ist, wobei der Kolben (1) mindestens zwei Punktegruppen aufweist, die jeweils einen auf der Außenkontur (7) angeordneten Auslegungspunkt (11), einen auf der Innenkontur (10) angeordneten Innenkonturpunkt (13) und den Referenzpunkt (9) aufweisen, wobei für jede der Punktegruppen gilt, dass der Abstand (12) von dem Auslegungspunkt (11) zu der geschlossenen Kurve gleich lang ist wie der Abstand (14) von dem Innenkonturpunkt (13) zu dem Referenzpunkt (9), dass der Auslegungspunkt (11), der Innenkonturpunkt (13) und der Referenzpunkt (9) auf einer Geraden liegen und dass der Innenkonturpunkt (13) zwischen dem Auslegungspunkt (11) und dem Referenzpunkt (9) liegt, wobei der Abstand (14) von dem Innenkonturpunkt (13) zu dem Referenzpunkt (9) für mindestens zwei der Punktegruppen unterschiedlich ist, wobei der Kolben (1) in dem Injektor (17) in einer Verschieberichtung längsverlagerbar gelagert ist und eingerichtet ist, mit der Kontaktfläche (15) voran in die Spitze (17) verschoben zu werden, wobei die geschlossene Kurve in ihrem Inneren eine Fläche begrenzt, die die gleiche Form hat wie ein zweiter innerer Querschnitt der Spitze (16) an deren engster Stelle, wobei die Normale des zweiten inneren Querschnitts parallel zu der Verschieberichtung ist.

3. Injektor gemäß Anspruch 1 oder 2, wobei der Kolben (1) mindestens drei der Punktegruppen aufweist.

4. Injektor gemäß einem der Ansprüche 1 bis 3, wobei die Außenkontur (7) eine von einem Kreis verschiedene Form hat.

5. Injektor gemäß einem der Ansprüche 1 bis 4, wobei die Innenkontur (10) eine Interpolation der Innenkonturpunkte (13) ist.

6. Injektor gemäß einem der Ansprüche 1 bis 5, wobei das Kissen (2) einen sich erweiternden Abschnitt (4) aufweist, dessen Querschnitt sich in Richtung zu der Kontaktfläche (15) hin erweitert.

7. Injektor gemäß Anspruch 6, wobei die Aussparung (6) sich in dem sich erweiternden Abschnitt (4) erstreckt und sich in dem sich erweiternden Abschnitt (4) in einer Richtung weg von der Kontaktfläche (15) verjüngt.

8. Injektor gemäß einem der Ansprüche 1 bis 7, wobei das Kissen (2) einen vorstehenden Abschnitt (3) aufweist, der quer zu einer Richtung, die parallel zu der Normalen der Kontaktfläche (15) ist, von dem verbliebenen Kissen (2) vorsteht und die Kontaktfläche (15) bildet.

9. Injektor gemäß einem der Ansprüche 2 bis 8, wobei der Referenzpunkt (9) so gewählt ist, dass der Referenzpunkt (9), wenn der Referenzpunkt (9) in die Verschieberichtung projiziert ist, mit dem Schwerpunkt eines ersten inneren Querschnitts der Spitze (16) zusammenfällt, dessen Normale parallel zu der Verschieberichtung ist.

10. Injektor gemäß einem der Ansprüche 1 und 3 bis 8, wobei der Kreis (8) den gleichen Durchmesser hat wie ein zweiter innerer Querschnitt der Spitze (16) an deren engster Stelle, wobei die Normale des zweiten inneren Querschnitts parallel zu der Verschieberichtung ist.

## Claims

1. Injector with a plunger (1) and a tip (16), wherein the injector (17) is configured to inject an intraocular lens (22) into the capsular bag of an eye, wherein the plunger (1) has a deformable cushion (2) which has an unsqueezed state and a squeezed state and, at one longitudinal end of the plunger (1), has a contact surface (15) into which a recess (6) is introduced, wherein in the unsqueezed state the contact surface (15) has an outer contour (7) externally delimiting the contact surface (15) and has an inner contour (10) externally delimiting the recess (6), wherein an imaginary reference point (9) is arranged in the recess (6), which reference point (9) is the center point of an imaginary circle (8) arranged in the plane of the contact surface (15), wherein the plunger (1) has at least two groups of points, which each have a design point (11), arranged on the outer contour (7), an inner contour point (13), arranged on the inner contour (10), and the reference point (9), it being the case for each of the groups of points that the distance (12) from the design point (11) to the circle (8) is the same length as the distance (14) from the inner contour point (13) to the reference point (9), that the design point (11), the inner contour point (13) and the reference point (9) lie on a straight line, and that the inner contour point (13) lies between the design point (11) and the reference point (9), wherein the distance (14) from the inner contour point (13) to the reference point (9) is different for at least two of the groups of points, wherein the plunger (1) is mounted in the injector (17) in such a way as to be displaceable longitudinally in a direction of displacement and is configured to be moved, with the contact surface (15) to the front, into the tip (17), wherein the reference point (9) is chosen such that, when the reference point (9) is projected in the direction of displacement, the reference point (9) coincides with the center of gravity of a first internal cross section of the tip (16), of which the normal is parallel to the direction of displacement.

2. Injector with a plunger (1) and a tip (16), wherein the injector (17) is configured to inject an intraocular lens (22) into the capsular bag of an eye, wherein the plunger (1) has a deformable cushion (2) which has an unsqueezed state and a squeezed state and, at one longitudinal end of the plunger (1), has a contact surface (15) into which a recess (6) is introduced, wherein in the unsqueezed state the contact surface (15) has an outer contour (7) externally delimiting the contact surface (15) and has an inner contour (10) externally delimiting the recess (6), wherein an imaginary reference point (9) is arranged in the recess (6), which reference point (9) is the center point of an imaginary closed curve arranged in the plane of the contact surface (15), wherein the plunger (1) has at least two groups of points, which each have a design point (11), arranged on the outer contour (7), an inner contour point (13), arranged on the inner contour (10), and the reference point (9), it being the case for each of the groups of points that the distance (12) from the design point (11) to the closed curve is the same length as the distance (14) from the inner contour point (13) to the reference point (9), that the design point (11), the inner contour point (13) and the reference point (9) lie on a straight line, and that the inner contour point (13) lies between the design point (11) and the reference point (9), wherein the distance (14) from the inner contour point (13) to the reference point (9) is different for at least two of the groups of points, wherein the plunger (1) is mounted in the injector (17) in such a way as to be displaceable longitudinally in a direction of displacement and is configured to be moved, with the contact surface (15) to the front, into the tip (17), wherein the closed curve in its interior delimits a surface area which has the same shape as a second internal cross section of the tip (16) at the narrowest point thereof, wherein the normal of the second internal cross section is parallel to the direction of displacement.

3. Injector according to Claim 1 or 2, wherein the plunger (1) has at least three of the groups of points.

4. Injector according to one of Claims 1 to 3, wherein the outer contour (7) has a shape different from a circle.

5. Injector according to one of Claims 1 to 4, wherein the inner contour (10) is an interpolation of the inner contour points (13).

6. Injector according to one of Claims 1 to 5, wherein the cushion (2) has a widening portion (4) whose cross section widens in the direction of the contact surface (15) .

7. Injector according to Claim 6, wherein the recess (6) extends in the widening portion (4) and tapers in the widening portion (4) in a direction away from the contact surface (15).

8. Injector according to one of Claims 1 to 7, wherein the cushion (2) has a protruding portion (3) which, transverse to a direction parallel to the normal of the contact surface (15), protrudes from the rest of the cushion (2) and forms the contact surface (15).

9. Injector according to one of Claims 2 to 8, wherein the reference point (9) is chosen such that, when the reference point (9) is projected in the direction of displacement, the reference point (9) coincides with the center of gravity of a first internal cross section of the tip (16), of which the normal is parallel to the direction of displacement.

10. Injector according to one of Claims 1 and 3 to 8, wherein the circle (8) has the same diameter as a second internal cross section of the tip (16) at the narrowest point thereof, wherein the normal of the second internal cross section is parallel to the direction of displacement.

## Revendications

1. Injecteur avec un piston (1) et une pointe (16), l'injecteur (17) étant adapté pour injecter une lentille intraoculaire (22) dans le sac capsulaire d'un oeil, le piston (1) présentant un coussinet déformable (2) qui a un état non écrasé et un état écrasé et qui présente une surface de contact (15) à une extrémité longitudinale du piston (1), dans laquelle est pratiqué un évidement (6), la surface de contact (15) présentant, à l'état non écrasé, un contour extérieur (7) délimitant extérieurement la surface de contact (15) et un contour intérieur (10) délimitant extérieurement l'évidement (6), un point de référence imaginaire (9) étant agencé dans l'évidement (6), lequel est le centre d'un cercle imaginaire (8) et agencé dans le plan de la surface de contact (15), le piston (1) présentant au moins deux groupes de points qui présentent chacun un point de conception (11) agencé sur le contour extérieur (7), un point de contour intérieur (13) agencé sur le contour intérieur (10) et le point de référence (9) ; pour chacun des groupes de points, la distance (12) du point de conception (11) au cercle (8) étant égale à la distance (14) du point de contour intérieur (13) au point de référence (9), le point de conception (11), le point de contour intérieur (13) et le point de référence (9) étant situés sur une ligne droite, et le point de contour intérieur (13) étant situé entre le point de conception (11) et le point de référence (9), la distance (14) du point de contour intérieur (13) au point de référence (9) étant différente pour au moins deux des groupes de points, le piston (1) étant monté dans l'injecteur (17) de manière à pouvoir se déplacer longitudinalement dans une direction de déplacement et étant adapté pour être déplacé dans la pointe (17) avec la surface de contact (15) en avant, le point de référence (9) étant choisi de telle sorte que, lorsque le point de référence (9) est projeté dans la direction de déplacement, le point de référence (9) coïncide avec le centre de gravité d'une première section transversale intérieure de la pointe (16), dont la normale est parallèle à la direction de déplacement.

2. Injecteur avec un piston (1) et une pointe (16), l'injecteur (17) étant adapté pour injecter une lentille intraoculaire (22) dans le sac capsulaire d'un oeil, le piston (1) présentant un coussinet déformable (2) qui a un état non écrasé et un état écrasé, et présentant une surface de contact (15) à une extrémité longitudinale du piston (1), dans laquelle est pratiqué un évidement (6), la surface de contact (15) présentant, à l'état non écrasé, un contour extérieur (7) délimitant extérieurement la surface de contact (15) et un contour intérieur (10) délimitant extérieurement l'évidement (6), un point de référence imaginaire (9) étant agencé dans l'évidement (6), lequel est le centre d'une courbe fermée imaginaire et agencée dans le plan de la surface de contact (15), le piston (1) présentant au moins deux groupes de points qui présentent chacun un point de conception (11) agencé sur le contour extérieur (7), un point de contour intérieur (13) agencé sur le contour intérieur (10) et le point de référence (9) ; pour chacun des groupes de points, la distance (12) du point de conception (11) à la courbe fermée étant égale à la distance (14) du point de contour intérieur (13) au point de référence (9), le point de conception (11), le point de contour intérieur (13) et le point de référence (9) étant situés sur une ligne droite, et le point de contour intérieur (13) étant situé entre le point de conception (11) et le point de référence (9), la distance (14) du point de contour intérieur (13) au point de référence (9) étant différente pour au moins deux des groupes de points, le piston (1) étant monté dans l'injecteur (17) de manière à pouvoir se déplacer longitudinalement dans une direction de déplacement et étant adapté pour être déplacé dans la pointe (17) avec la surface de contact (15) en avant, la courbe fermée délimitant à l'intérieur de celle-ci une surface ayant la même forme qu'une deuxième section transversale intérieure de la pointe (16) à son point le plus étroit, la normale de la deuxième section transversale intérieure étant parallèle à la direction de déplacement.

3. Injecteur selon la revendication 1 ou 2, dans lequel le piston (1) présente au moins trois des groupes de points.

4. Injecteur selon l'une quelconque des revendications 1 à 3, dans lequel le contour extérieur (7) a une forme autre qu'un cercle.

5. Injecteur selon l'une quelconque des revendications 1 à 4, dans lequel le contour intérieur (10) est une interpolation des points de contour intérieur (13).

6. Injecteur selon l'une quelconque des revendications 1 à 5, dans lequel le coussinet (2) présente une section s'élargissant (4) dont la section transversale s'élargit en direction de la surface de contact (15).

7. Injecteur selon la revendication 6, dans lequel l'évidement (6) s'étend dans la section s'élargissant (4) et se rétrécit dans la section s'élargissant (4) dans une direction s'éloignant de la surface de contact (15).

8. Injecteur selon l'une quelconque des revendications 1 à 7, dans lequel le coussinet (2) présente une section en saillie (3) qui fait saillie à partir du coussinet restant (2) transversalement à une direction parallèle à la normale à la surface de contact (15) et qui forme la surface de contact (15).

9. Injecteur selon l'une quelconque des revendications 2 à 8, dans lequel le point de référence (9) est choisi de telle sorte que, lorsque le point de référence (9) est projeté dans la direction de déplacement, le point de référence (9) coïncide avec le centre de gravité d'une première section transversale intérieure de la pointe (16), dont la normale est parallèle à la direction de déplacement.

10. Injecteur selon l'une quelconque des revendications 1 et 3 à 8, dans lequel le cercle (8) a le même diamètre qu'une deuxième section transversale intérieure de la pointe (16) à son point le plus étroit, la normale de la deuxième section transversale intérieure étant parallèle à la direction de déplacement.
